# EUROPEAN PATENT APPLICATION

(11) **EP 1 933 136 A1**
(43) Date of publication of application: **18.06.2008**
(21) Application number: 06126058.4
(22) Date of filing: 13.12.2006
(51) Int. Cl.: G01N 33/50, G01N 33/68

(54) **High throughput screen (HTS) to identify chemotherapeutic compounds by interfering with cell cycle checkpoint functions**

(71) Applicant: Novartis AG, 4056 Basel (CH)
(72) Inventor: Movva, Rao, 4102 Binningen (CH); Gasser, Susan M., 1012 Lausanne (CH); Filipuzzi, Ireos, 4058 Basel (CH)
(74) Representative: Bourgarel, Denis Jacques Marie

(57) **Abstract**

The present invention relates to a method for the identification of synergistic compounds which act synergistically with compounds blocking DNA replication for e.g. the treatment of cancer, said method comprising the steps of a) incubating a compound of interest with a mutant yeast strain, wherein said mutant yeast strain is deficient for an orthologue of a human protein involved in check point control, and wherein the growth medium of said mutant yeast strain comprises a compound blocking DNA replication at a concentration which is non-toxic for said mutant yeast strain, and b) incubating said compound of interest with the corresponding wild type yeast strain of the mutant yeast strain of step a), wherein the growth medium of said wild-type yeast strain comprises a compound blocking DNA replication at a concentration which is non-toxic for said wild-type yeast strain, wherein a compound of interest which inhibits the growth of said mutant yeast strain in the presence of a compound blocking DNA replication at a concentration which is non-toxic for said mutant yeast strain, but does not inhibit the growth of the corresponding wild-type yeast strain in the presence of a compound blocking DNA replication at a concentration which is non-toxic for said wild type-yeast strain is a synergistic compounds which act synergistically with compounds blocking DNA replication for e.g. the treatment of cancer.

## Description

### Field of the invention

The present invention relates to i) the concept of identifying novel chemotherapeutic agents by interfering with S-phase cell cycle checkpoint functions and ii) the development and application of a high throughput screen (HTS) to identify chemotherapeutic compounds by interfering with S-phase cell cycle checkpoint functions.

### Background of the invention

A universal hallmark of cancer is high genetic instability caused by replication defects and/or impaired DNA damage response. The prevention of genetic instability, and thus cancer evolution, depends therefore on an intact replication checkpoint pathway (reviewed in Nakanishi et al, 2006). Mutations in genes with checkpoint function have been found to lead to a pre-disposition to cancer (e.g. ATR (O'Driscoll et al, 2003), BLM/WRN/RECQ4 (reviewed in Mohaghegh and Hickson, 2001)). Tumor cells are found to be highly sensitive to disturbance of DNA replication. Therefore, common cancer treatments (e.g. Hydroxyurea (HU), Adriamycin, Camptothecin, etc ...) are based on blocking DNA replication. A drawback of such an intervention is the narrow therapeutic window of most DNA replication blockers. There is thus a need for compounds which would synergistically influence the effect of said inhibitors and thereby increasing the therapeutic window of said DNA replication blockers. Such compounds would be useful for the treatment of cancers and solid tumors, particularly all those that cannot be removed by surgery and thus are being treated by standard chemotherapies.
The inhibition of S-phase checkpoint functions by a chemical or natural compound would make cancer cells even more susceptible to replication blockers as this additional perturbation would drive the treated cells into apoptosis. These compounds would therefore allow combinational therapies at reduced chemotherapeutic concentrations which would increase the efficiency of such agents and decrease the adverse side effects.

### Summary of the invention

The inventors have discovered a simple and robust method for the identification of compounds fulfilling the above-described needs. The present invention thus encompasses a method for the identification of compounds which act synergistically with compounds blocking DNA replication for e.g. the treatment of cancer, said method comprising the steps of a) incubating a compound of interest with a mutant yeast strain, wherein said mutant yeast strain is deficient for an orthologue of a human protein involved in check point control, and wherein the growth medium of said mutant yeast strain comprises a compound blocking DNA replication at a concentration which is non-toxic for said mutant yeast strain, and b) incubating said compound of interest with the corresponding wild type yeast strain of the mutant yeast strain of step a), wherein the growth medium of said wild-type yeast strain comprises a compound blocking DNA replication at a concentration which is non-toxic for said wild-type yeast strain, wherein a compound of interest which inhibits the growth of said mutant yeast strain in the presence of a compound blocking DNA replication at a concentration which is non-toxic for said mutant yeast strain, but does not inhibit the growth of the corresponding wild-type yeast strain in the presence of a compound blocking DNA replication at a concentration which is non-toxic for said wild type-yeast strain is a synergistic compounds which act synergistically with compounds blocking DNA replication for the treatment of various diseases influenced by the cell cycle checkpoint control (e.g. cancer). Preferably, the compound blocking DNA replication is selected from the group consisting of busulfan, cisplatin, carboplatin, chlorambucil, cyclophosphamide, ifosfamide, dacarbazine, mechlorethamine (nitrogen mustard), melphalan, temozolomide, streptozocin, carmustine, lomustine, fluorouracil, capecitabine, 6-mercaptopurine, methotrexate, gemcitabine, cytarabine, fludarabine, pemetrexed, daunorubicin, doxorubicin, epirubicin, idarubicin, mitoxantrone, topotecan, irinotecan, etoposide, teniposide, L-asparaginase, hydroxyurea, dactinomycin, thalidomide, and tretinoin. Most preferably, the compound blocking DNA replication is hydroxyurea (HU).
In one particular embodiment, the concentration of said compound (e.g. HU) blocking DNA replication is between 2 and 15 mM, preferably between 4 and 10 mM, more preferably about 5 mM.
In another particular embodiment, the orthologue of a human protein involved in checkpoint control is selected from the group consisting of TEL1, MEC1, DDC2/LCD1, SGS1, RAD9, MIH1, CDC7, CHK1, RAD53, DBF4, MEC3, MRC1, MRE11, XRS2, RAD17, RAD24, RAD50, DDC1, RFC4, SCC1, PDS1, ESP1, SMC1, TOF1, DPB11 and SRS2.

### Description of the figures

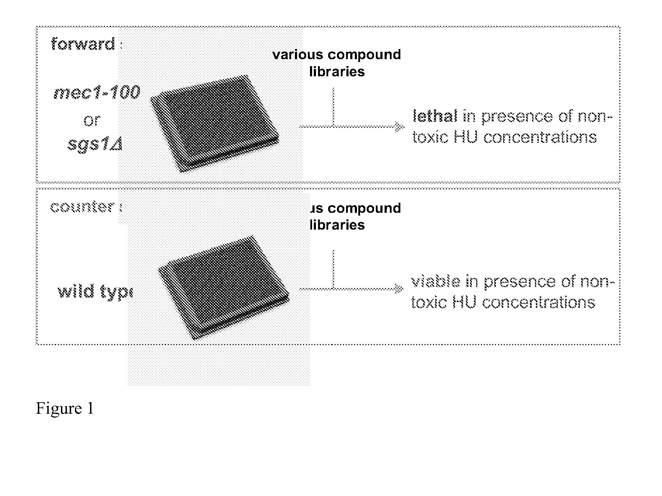

Figure 1: Concept of the assay illustrated in the Examples: The assay is based on yeast growth inhibition using the mutant yeast strains *mec1-100* and sgs*1*□ (forward screen) and the corresponding wild type yeast strain (counter screen) to eliminate nonspecific yeast growth inhibitors. Compounds inhibiting the mutants, but not the wild type cell line are considered specific.

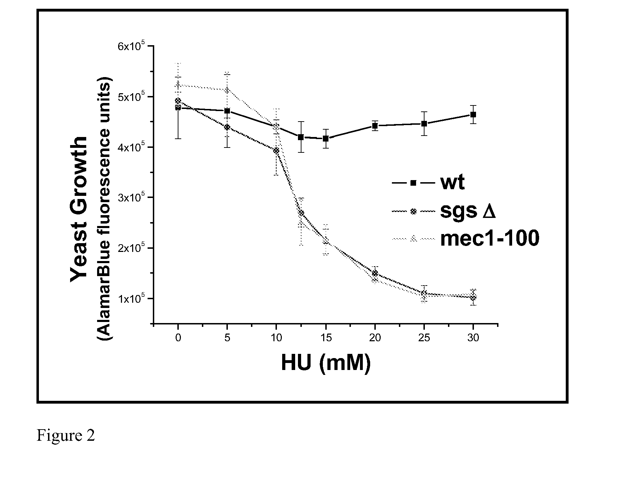

Figure2: HU sensitivity: To show that the mutant yeast cell lines, but not the wild type, are sensitive to HU, dose-response curves were performed. As expected, the mutant cell lines were found to have an HU hypersensitive phenotype, allowing the identification of cancer cells specific synergistic compounds.

### Detailed description of the invention

The approach taken by the present inventors has been to elaborate a simple and robust method to find compounds which, by interfering with replication checkpoints, increase genomic instability of cancer cells and thus enhance efficiency of standard chemotherapeutics by pushing cancer cells selectively into apoptosis.

The inventors have therefore chosen to provide a screening system based on the yeast model as checkpoint pathways are highly conserved in evolution. For example, the yeast *S*. *cerevisiae* has an ATR orthologue, Mec1p, and a BLM orthologue, Sgs1 p. The yeast *MEC1*/*SGS1* system was used to elucidate the DNA replication fork complex and S-phase checkpoint function of ATR and BLM. Interestingly, the mutation *mec1-100* (F1179S and N1700S) shows impaired S-phase specific checkpoint function, but intact G2M checkpoint activity, allowing to dissect the two activities of Mec1p (Cobb et al, 2006 and references therein). *mec1-100* and *sgs1Δ* phenotypes coincides with tumor cells mutated in the ATR and BLM orthologues (reduced DNA damage response, replication fork collapse, enhanced chromosome instability and impaired survival on HU). Therefore the *mec1-100* and sgs*1*Δ yeast strains typically represent an attractive model for cancer cells with impaired DNA damage response. Such a system can be used to screen for anti-cancer agents in combination with common cancer treatments (e.g. Hydroxyurea (HU) or others mentioned above) based on blocking DNA replication. In addition to the well-defined biological mechanism, the yeast system also allows the set-up of a robust assay which is amenable to miniaturization and automatisation allowing high throughput compound screening. In contrast, as compared to yeast, mammalian BLM and/or ATR deficient cell lines are fragile and thus not amenable to HTS.

An exemplary assay of the invention is the Mec1p and Sgs1 p assays illustrated in the examples. The PI3-K like kinase ATR is a DNA damage sensor involved in checkpoint control (S and G2/M) and damage repair. The RecQ helicase BLM plays an important role in S-phase checkpoint and is involved in maintaining genomic integrity during S-phase. Mutations in ATR and BLM lead to phenotypes coinciding with those of tumor cells (e.g. reduced DNA damage response, increased inter- and intra- chromosomal recombination, enhanced chromosome mis-segregation, abnormal profile of DNA replication intermediates, reduced ability to induce apoptosis and increased sensitivity to genotoxic agents). The present inventors have found out that cells with impaired S-phase checkpoint function can be used as models for cancer cells which are extremely well suited for the identification of synergistic compounds as described herein.

As explained above, checkpoint pathways are highly conserved in evolution. The yeast *S*. *cerevisiae* has an ATR orthologue, Mec1p, and a BLM orthologue, Sgs1 p. Human orthologues have been shown to functionally complement yeast gene deletions. The yeast *MEC1*/*SGS1* system was used to elucidate the DNA replication fork complex and S-phase checkpoint function of ATR and BLM. Interestingly, the mutation *mec1-100* (F1179S and N1700S) shows impaired S-phase specific checkpoint function, but intact G2M checkpoint activity, allowing to dissect the two activities of Mec1p. *mec1-100* and *sgs1Δ* phenotypes coincides with tumor cells mutated in the ATR and BLM orthologues (reduced DNA damage response, replication fork collapse, enhanced chromosome instability and impaired survival on HU). Therefore the *mec1-100* and *sgs1Δ* yeast strains represent an attractive model for cancer cells with impaired DNA damage response. This system can be used to screen for anti-cancer agents in combination with chemotherapeutics like, for example, HU.

It will be however immediately evident to the skilled person that the above system is only exemplary and that any system wherein a yeast orthologue of a gene known to be involved in cancer, or a gene which product is known to be a target of anticancer treatments, is present, can be used similarly. Such alternative system include but are not limited to genes regulating fundamental cellular processes like checkpoint control or cell cycle control. Examples of such genes are represented by the family of cyclins, cyclin-dependent kinases (CDKs), by the mitogen-activated protein kinase (MAPK) signaling cascade and other genes with regulatory functions. Examples of genes involved in checkpoint control can be found in Table 1.

**Table 1: A list of exemplary human protein involved in checkpoint control and their yeast (S. cerevisiae) orthologues.**

| ***S. cerevisiae*** | ***H. sapiens*** |
|---|---|
| *TEL1* | ATM |
| *MEC1* | ATR |
| *DDC2*/*LCD1* | ATRIP |
| *SGS1* | BLM |
| *RAD9* | BRCA1 |
| *MIH1* | Cdc25 |
| *CDC7* | CDC7 |
| *CHK1* | CHK1 |
| *RAD53* | CHK2/CDS1 |
| *DBF4* | DBF4/ASK |
| unknown | DNA-PK-Ku |
| *MEC3* | HUS1 |
| unknown | MDC1 |
| *MRC1* | MRC1, claspin |
| *MRE11* | MRE11 |
| *XRS2* | NBS1 |
| unknown | p53 |
| *RAD17* | RAD1 |
| *RAD24* | RAD17 |
| *RAD50* | RAD50 |
| *DDC1* | RAD9 |
| *RFC4* | RFC2-5 |
| *SCC1* | SCC1 |
| *PDS1* | Securin |
| *ESP1* | Separase |
| *SMC1* | SMC1 |
| *TOF1* | TIM |
| *DPB11* | TopBP1, p53BP |
| *SRS2* | unknown |

The expression "yeast cell" is used herein to mention unicellular fungi of the phylum Ascomycota that reproduce by fission or budding and are capable of fermenting carbohydrates into alcohol and carbon dioxide. Preferably said yeast cells are *Saccharomycetaces* or *Schizossaccharomycefales*, such as *Saccharomyces pombe, Saccharomyces cerevisiae, Candida* or *Pichia.* Most preferably yeast cells of the species *Saccharomyces cerevisiae* are used for manipulation in accordance with the present invention. A genetically stable yeast cell is also referred to as a yeast strain.

A "mutant" refers to an organism which, as a result of either natural processes or mutagenesis techniques including but not limited to site directed mutagenesis, contains one or more mutations (deletions, insertions and/or substitutions) in its genome as compared to the wild-type strain (used for its taxonomic classification), which mutation(s) leads to an inactivation of the target. Preferred embodiments of such mutant according to the present invention are deletion yeast strains, more particularly, deletion strains comprising one or more deletions in one or more genes coding for the target, resulting in the strain lacking a functional gene for the target or a functional target. A functional gene, as used herein refers to a gene which is capable of expressing its natural gene product.

In the context of the present invention, yeast strains "lacking one or more functional genes" are yeast strains in which one or more genes cannot be expressed (no gene product) or which express an aberrant gene product, i.e. a product which is not the natural gene product. This definition also applies *mutatis mutandis* for any non-human organism. Preferably, according to the present invention, such an aberrant gene product, is a gene product which has lost its original function (as present in the wild-type strain). Alternatively such aberrant gene product has a reduced function (less than 50%, less than 75%, or even less than 90%) when compared to the wild type function (enzymatic activity, binding affinity etc.). Yeast cells or strains lacking one or more functional genes can be the result of different factors, such as, but not limited to one or more mutations in the gene itself or in a gene encoding a factor (protein, RNA, dsRNA) which is capable of modifying the expression of said gene, a truncation (caused e.g. by aberrant expression of an enzyme capable of degrading said protein); the complete absence of a gene; the insertion of a transposon; expression of a transgene (resulting in missense, antisense, sense expression) which modifies the expression or the activity of the natural gene product.

As used herein, the expression "common cancer treatment" refers to treatment based on interfering with DNA damage repair and DNA replication and other S-phase functions of the cell cycle. Such agents include alkylating agents (like busulfan, cisplatin, carboplatin, chlorambucil, cyclophosphamide, ifosfamide, dacarbazine, mechlorethamine (nitrogen mustard), melphalan, and temozolomide), nitrosoureas (like streptozocin, carmustine, and lomustine), antimetabolites (like fluorouracil, capecitabine, 6-mercaptopurine, methotrexate, gemcitabine, cytarabine, fludarabine, and pemetrexed, anthracyclines and related drugs (like daunorubicin, doxorubicin, epirubicin, idarubicin, and mitoxantrone), topoisomerase inhibitors (like topotecan, irinotecan, etoposide and teniposide) and others (like L-asparaginase, hydroxyurea, dactinomycin, thalidomide, and tretinoin).

The term "inhibition" is used herein to denote the activity of a compound which by interfering with its target, directly or indirectly, influences fundamental processes (like the cell cycle, checkpoint control or others and others) leading to reduced growth of the treated cells. Reduced growth is defined by the potential of a compound to completely or partially stop or slow down proliferation of a cell. Growth inhibition can be determined by several state of the art techniques monitoring cell number. These include measuring turbidity of a cell culture or using fluorescent, chromophoric or radioactive probes reflecting the number of living cells in a given sample. Growth inhibition of a test compound is determined by a statistically significant reduction in cell number, compared to its corresponding placebo control. A statistically significant growth reduction can be usually determined for a inhibition range of 10% to 100%.

The term "compound" is used herein to denote a chemical structure, a mixture of chemical structures, a peptide, a biological substance such as a macromolecule, or an extract made from biological materials such as bacteria, plants, fungi, or animal (particularly mammalian) cells or tissues. Preferred embodiments are chemical structures and a mixture of chemical structures. According to one aspect of the present invention, methods are provided for identifying compounds capable of influencing, modifying (increasing or decreasing) the phenotypic effect of the target gene or protein in yeast. The influence of a compound on the phenotypic effect observed in yeast cells can be direct, by interacting with the target, or can be indirect, by affecting the expression of the target protein in yeast, or by affecting inhibitors or enzymes which themselves influence the phenotypic effect of the target. Alternatively, the effect of these compounds may be by interacting with proteins or enzymes which directly mediate the phenotypic enact of the target. Such compounds include but are not limited to proteins or polypeptides (such as heat shock proteins receptors, enzymes, ligands, nuclear or cytoplasmic proteins, chaperones, hormones, antigens, growth factors, regulatory proteins, structural proteins) nucleic acids (RNAs, ribozymes, DNAs or cDNAs) or chemical compounds. The screening of compounds from natural or synthetic libraries is also envisaged.

A screening method, as used herein refers to a method for identifying specific compounds. More particularly, the screening method of the present invention allows the identification of compounds based on their ability to increased the therapeutic window of common cancer treatments.

### Examples

The illustrative assay disclosed herein is based on yeast growth inhibition using the mutant yeast strains mentioned above, i.e. *mec1-100* and *sgs1Δ,* and the corresponding wild type yeast strain to eliminate nonspecific yeast growth inhibitors (see figure 1). Compounds inhibiting the mutants, but not the wild type cell line are considered specific.

### Assay protocol:

Inoculate 2 milliliters YPD medium with the corresponding yeast strains and incubate the cultures over night at 30°C with constant shaking at 170 rpm. The next day, use 250 micro-liters of the over night culture to inoculate 100 milliliters SD medium supplemented with Uracil (20 mg/l), Leucine (10 mg/l), Histidine (20 mg/l), Adenine (20 mg/l) and Tryptophan (20 mg/l). Incubate at 30°C constantly shaking at 170 rpm until an OD600 of 0.8 - 1.0 is reached. Then dilute back the cultures to an OD600 of 0.01 and add 5 µl of these cultures into the 1536 well assay plates containing the test compounds at a final concentration of 20 micro-molar. Further incubate for 16 hours at 30°C in humid atmosphere (95% humidity). After 16 hours add 2.4 micro-liters of 150 mM KPi pH6.4, Alamar Blue 4.5% (v/v) and further incubate for 3 hours at 30°C in 95% humidity. Read plates in a standard plate reader (e.g. Viewlux or EnVision) using an emission wavelength of 560 nano-meters and an excitation wavelength of 590 nano-meters.

The two assays described herein-above were developed, down-scaled to the 1536 well format and used to test a set of 20'000 compounds at a final compound concentration of 20 micro-molar. The screens were found to be sensitive (H/L ~10) and robust (z' > 0.8) and lead to the identification of 9 compounds (out of 20'000) with significant selectivity compared to wild type cells.

### References

Cobb JA, Schleker T, Rojas V, Bjergbaek L, Tercero JA, Gasser SM (2006). Replisome instability, fork collapse, and gross chromosomal rearrangements arise synergistically from Mec1 kinase and RecQ helicase mutations. Genes Dev. 2005 Dec 15;19(24):3055-69.

Mohaghegh P, Hickson ID. (2001). DNA helicase deficiencies associated with cancer predisposition and premature ageing disorders. Hum Mol Genet. 2001 Apr;10(7):741-6.

Nakanishi M, Shimada M, Niida H. (2006). Genetic instability in cancer cells by impaired cell cycle checkpoints. Cancer Sci. 2006 Oct;97(10):984-9.

O'Driscoll M, Ruiz-Perez VL, Woods CG, Jeggo PA, Goodship JA. (2003). A splicing mutation affecting expression of ataxia-telangiectasia and Rad3-related protein (ATR) results in Seckel syndrome. Nat Genet. 2003 Apr;33(4):497-501.

## Claims

1. A method for the identification of synergistic compounds which act synergistically with compounds blocking DNA replication for e.g. the treatment of cancer, said method comprising the steps of
a) incubating a compound of interest with a mutant yeast strain, wherein said mutant yeast strain is deficient for an orthologue of a human protein involved in check point control, and wherein the growth medium of said mutant yeast strain comprises a compound blocking DNA replication at a concentration which is non-toxic for said mutant yeast strain, and
b) incubating said compound of interest with the corresponding wild type yeast strain of the mutant yeast strain of step a), wherein the growth medium of said wild-type yeast strain comprises a compound blocking DNA replication at a concentration which is non-toxic for said wild-type yeast strain,
wherein a compound of interest which inhibits the growth of said mutant yeast strain in the presence of a compound blocking DNA replication at a concentration which is non-toxic for said mutant yeast strain, but does not inhibit the growth of the corresponding wild-type yeast strain in the presence of a compound blocking DNA replication at a concentration which is non-toxic for said wild type-yeast strain is a synergistic compounds which act synergistically with compounds blocking DNA replication for e.g. the treatment of cancer.

2. The method of claim 1 wherein the compound blocking DNA replication is selected from the group consisting of busulfan, cisplatin, carboplatin, chlorambucil, cyclophosphamide, ifosfamide, dacarbazine, mechlorethamine (nitrogen mustard), melphalan, temozolomide, streptozocin, carmustine, lomustine), fluorouracil, capecitabine, 6-mercaptopurine, methotrexate, gemcitabine, cytarabine, fludarabine, and pemetrexed, daunorubicin, doxorubicin, epirubicin, idarubicin, mitoxantrone, topotecan, irinotecan, etoposide, teniposide, L-asparaginase, hydroxyurea, dactinomycin, thalidomide, and tretinoin

3. The method of claims 1 or 2 wherein the compound blocking DNA replication is hydroxyurea (HU).

4. The method of claims 1-3 wherein the concentration of the compound blocking DNA replication is between 2 and 15 mM, preferably between 4 and 10 mM, more preferably about 5 mM.

5. The method of claims 1-4 wherein the orthologue of a human protein involved in checkpoint control is selected from the group consisting of TEL1, MEC1, DDC2/LCD1, SGS1, RAD9, MIH1, CDC7, CHK1, RAD53, DBF4, MEC3, MRC1, MRE11, XRS2, RAD17, RAD24, RAD50, DDC1, RFC4, SCC1, PDS1, ESP1, SMC1, TOF1, DPB11 and SRS2.
